# EUROPEAN PATENT APPLICATION

(11) **EP 2 436 664 A1**
(43) Date of publication of application: **04.04.2012**
(21) Application number: 10774843.6
(22) Date of filing: 30.04.2010
(51) Int. Cl.: C07C 7/12, C07C 7/148, C07C 9/15, C07C 9/21, C07C 15/073, C07C 15/24, C07C 17/389, C07C 17/395, C07C 25/02, C07C 25/10, C07C 25/22

(54) **METHOD FOR PURIFYING ORGANIC SOLVENT**

(30) Priority: 12.05.2009 JP 2009115856
(71) Applicant: Wako Pure Chemical Industries, Ltd., Osaka-shi Osaka 540-8605 (JP)
(72) Inventor: KUROOKA, Masaharu, Kawagoe-shi Saitama 350-1101 (JP); ITOH, Tomohiro, Kawagoe-shi Saitama 350-1101 (JP); MAKINO, Yuichi, Kawagoe-shi Saitama 350-1101 (JP)
(74) Representative: Winter, Brandl, Fürniss, Hübner, Röss, Kaiser, Polte - Partnerschaft
(86) International application number: PCT/JP2010/057663
(87) International publication number: WO 2010/131585

(57) **Abstract**

The present invention relates to a method for purifying a compound selected from a compound represented by the following general formula [1] or [2], a saturated aliphatic hydrocarbon having no substituent, or kerosene: 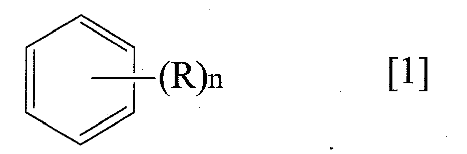 (R represents a halogen atom or a lower alkyl group having 1 to 3 carbon atoms, and n represents an integer of 1 to 5); 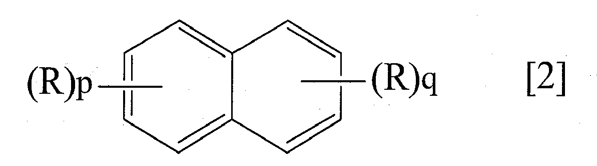 (R is the same as above, p and q independently represent an integer of 0 to 4, with the proviso that at least one ofp and q is not 0), comprising:
removing a impurity contained in said compound by
a first step comprising contacting said compound with ozone gas, and then
a second step comprising contacting said compound with an adsorbent selected from zeolite or a basic adsorbent.

## Description

### TECHNICAL FIELD

The present invention relates to a novel purification method of an organic solvent.

### BACKGROUND ART

Aromatic compounds such as trichlorobenzene which are used as a mobile phase solvent for high performance liquid chromatography (HPLC) are desired to be the one which is highly pure not containing any impurities to influence subsequent measurements.

However, some of aromatic compounds contain impurities having fluorescence such as chlorophenol and dichlorophenol, and previously a treatment to remove fluorescent substances has been carried out using an adsorbent such as zeolite. However, this method needs to use a large amount of zeolite. In particular, concerning trichlorobenzene, it sometimes contains polychlorobiphenyl (PCB) which is a harmful substance, in addition to fluorescent substances. Therefore, when trichlorobenzene contaminated with PCB is treated with a large amount of zeolite, a large amount of used zeolite contaminated with PCB is produced, and a step to treat this large amount of used zeolite was additionally required.

Therefore, it is present situation that establishment of a purification method of an aromatic compound such as trichlorobenzene which does not require use of a large amount of zeolite and is capable of removing the above-described impurities simply and effectively has been desired.

As a method for removing impurities such as fluorescent substance from aromatic compounds, distillation method is also known. However, when a compound having a high boiling point is purified by distillation method, cumbersome operations such as distillation under reduced pressure are required. In addition, when a compound having a benzene ring in a structure thereof is distilled, there is a risk that the still kettle is contaminated with the compound. Further, special facilities are needed to carry out the purification method by distillation, and this is also a problem from the viewpoint of cost performance.

Several methods where impurities in an organic solvent including aromatic compound are removed without carrying out distillation have been known until now. Such methods include, for example,
(1) a method for purifying an aromatic nitro-compound, wherein a small amount of sulfur compound contained in an aromatic nitro-compound is oxidatively decomposed by ozone treatment, and then removed by washing with an aqueous alkali solution (Patent Literature 1);
(2) a method for removing a sulfur compound from an aromatic compound, wherein a small amount of sulfur compound contained in an aromatic compound such as benzene is oxidatively decomposed by contacting with ozone, and then removed by washing with an aqueous alkali solution (Patent Literature 2).
   However, in both of the above-described methods, since raw material is treated with ozone, and then impurities are removed by washing with aqueous alkali solution, that is, purification operation by a liquid - liquid extraction method is carried out, there are problems that facilities for carrying out stirring, liquid separation, and the like are needed, and also recovery ratio of purified product is not high enough.

In addition, organic solvents other than aromatic compound also sometimes contain impurities such as fluorescent substance or UV-absorbing substance, and several purification methods have been known, including:
(1) a method for purifying acetonitrile by obtaining acetonitrile as a by-product in producing acrylonitrile by ammonia oxidation reaction of propylene or isobutylene in the presence of a catalyst, pretreating acetonitrile to obtain semi-purified acetonitrile containing reduced amount of impurities, contacting further the semi-purified acetonitrile with a gas containing ozone, neutralizing with a basic substance, and then distilling (Patent Literature 3);
(2) a method for purifying acetonitrile by obtaining acetonitrile as a by-product in producing acrylonitrile by ammonia oxidation reaction of propylene or isobutylene in the presence of a catalyst, contacting acetonitrile with sulfuric acid, then separating sulfuric acid part, subsequently contacting with a gas containing ozone, then distilling (Patent Literature 4); and
(3) a method for purifying crude acetonitrile by contacting crude acetonitrile with nascent oxygen (ozone), then contacting with basic substance, adsorbent, and the like, subsequently separating and removing a substance reducing permanganate formed by the ozone treatment, and further separating and removing low-boiling substance or high-boiling substance by distillation method or membrane separation method (Patent Literature 5); and the like.

However, when crude acetonitrile is treated with ozone, many kinds of by-products are formed. Therefore, the above-described method (1) essentially requires such operations as treating crude acetonitrile with ozone, then basic substance, and after that further distilling.

The above-described method (2) also needs such operations as contacting crude acetonitrile formed as a by-product with sulfuric acid, then ozone, and after that further distilling.

Furthermore, in the above-described method (3), purified by-product cannot be removed only by contacting the ozone-treated semi-purified acetonitrile with a basic substance, adsorbent, and the like. Therefore, a step to separate and remove a substance reducing permanganate and a step to separate and remove low-boiling point substance and high-boiling point substance by distillation method or membrane separation method are further needed.

In addition, for example, n-heptane is purified at present through treatments by zeolite F-9 column, sulfuric acid, distillation, and further zeolite F-9 column again in this order. However, there are many problems, for example, operation is cumbersome; a large volume of washing wastewater is obtained; quality varies lot to lot; and the like.

That is, in any method of previously known method, there were such problems that a cumbersome purification step had to be done and that special facilities etc. were required for purification.

Furthermore, recent years, kerosene is expected to be used as a fuel for household fuel battery. However, a small amount of sulfur compound has been mixed in the commercially available kerosene, and it has been known that this impairs catalyst performance of the fuel battery. For this reason, a method for efficiently removing the sulfur compound in kerosene has been also desired.

### CITATION LIST

### PATENT LITERATURE

Patent Literature 1: JP-A-01-011626
Patent Literature 2: JP-A-06-018791
Patent Literature 3: JP No. 3104312
Patent Literature 4: JP No. 3001020
Patent Literature 5: JP No. 2989477

### SUMMARY OF INVENTION

### PROBLEM TO BE SOLVED BY THE INVENTION

The present invention is directed to establish a purification method okf an organic solvent, which is capable of purifying in high purity an organic solvent containing impurities such as UV-absorbing substance, fluorescent substance, and sulfur compound, as well as reducing an amount of zeolite etc. to be used for purification.

### MEANS FOR SOLVING THE PROBLEM

The inventors of the present invention have intensively studied a way to solve the above-described problem. As a result, the inventors have found that by contacting an organic solvent with ozone gas before treating with zeolite which is one of the conventionally used purification methods of organic solvents, an organic solvent of high purity can be obtained by efficiently removing impurities in the organic solvent as well as reducing dramatically an amount of zeolite to be used, and completed the present invention.

That is, the present invention provides a method for purifying a compound selected from a compound represented by the following general formula [1] or [2], a saturated aliphatic hydrocarbon having no substituent, or kerosene: 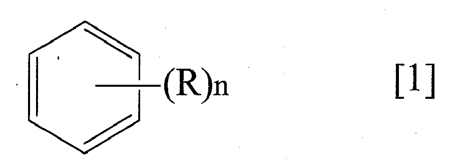 (R represents a halogen atom or a lower alkyl group having 1 to 3 carbon atoms, and n represents an integer of 1 to 5); 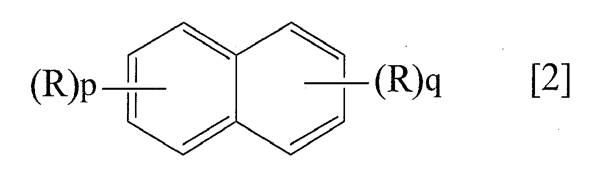 (R is the same as above, p and q independently represent an integer of 0 to 4, with the proviso that at least one of p and q is not 0), comprising:
removing a impurity contained in the compound by
   a first step comprising contacting said compound with ozone gas, and then
   a second step comprising contacting said compound with an adsorbent selected from zeolite or a basic adsorbent.

### EFFECT OF THE INVENTION

According to the purification method of the present invention, an organic solvent containing impurities such as UV-absorbing substance, fluorescent substance, and sulfur compound can be purified in high purity without requiring further distillation operation. In addition, by the purification method of the present invention, an amount of zeolite to be used can be reduced to a much smaller amount compared with the amount previously used.

### BRIEF DESCRIPTION OF THE DRAWINGS

Fig. 1 shows a UV absorption spectrum of the simulated kerosene obtained in Example 8.
Fig. 2 shows a UV absorption spectrum of a purified product of the simulated kerosene obtained in Example 8.
Fig. 3 shows a three-dimensional fluorescence spectrum of the simulated kerosene obtained in Example 8.
Fig. 4 shows a three-dimensional fluorescence spectrum of a purified product of the simulated kerosene obtained in Example 8.

### EMBODIMENTS FOR CARRYING OUT THE INVENTION

In the purification method of the present invention, a solvent to be purified is an organic solvent which does not react with ozone gas to be used in the first step and is in a liquid state at ordinary temperature (any temperature from 0°C to 40°C). Specifically, it is the compound represented by the general formula [1] or the general formula [2] or the saturated aliphatic hydrocarbon having no substituent, relevant to the present invention as described above.

In the compound represented by the general formula [1] or [2] relevant to the present invention, the halogen atom represented by R includes a fluorine atom, a chlorine atom, a bromine atom, an iodine atom, and the like.

In the compound represented by the general formula [1] or [2] relevant to the present invention, the lower alkyl group having 1 to 3 carbon atoms represented by R includes a methyl group, an ethyl group, and a n-propyl group.

Specific example of the compound represented by the general formula [1] relevant to the present invention includes trichlorobenzene, chlorobenzene, ethylbenzene, and the like. Among them, trichlorobenzene is preferable.

Specific example of the compound represented by the general formula [2] relevant to the present invention includes 1-chloronaphthalene, 1-methylnaphthalene, 2-methylnaphthalene, and the like. Among them, 1-chloronaphthalene and 1-methylnaphthalene are preferable.

The saturated aliphatic hydrocarbon having no substituent relevant to the present invention includes all of such compound, so long as it is in a liquid state at ordinary temperature (any temperature from 0°C to 40°C). The hydrocarbon may be any one of linear, branched or cyclic one, and has 4 to 20 carbon atoms, preferably 4 to 15 carbon atoms, and more preferably 4 to 12 carbon atoms. Specifically, the hydrocarbon includes, for example, cyclobutane, 2-methylbutane, 2,2-dimethylpropane (neopentane), n-pentane, 2-methylbutane, cyclopentane, methylcyclobutane, n-hexane, n-hexane, 2-methylpentane, 3-methylpentane, 2-methylpentane, n-hexane, 2-ethylbutane, 2-ethylbutane, 2-methylpentane, 3-methylpentane, 2-methylpentane, 2,2-dimethylbutane, 2,3-dimethylbutane, 2,2-dimethylbutane, n-hexane, 2-methylpentane, 2-methylpentane, 2,3-dimethylbutane, 2-methylpentane, 2,2-dimethylbutane, 3-methylpentane, 2,3-dimethylbutane, 2,2-dimethylbutane, cyclohexane, methylcyclopentane, n-heptane, n-heptane, 2,4-dimethylpentane, 2,3-dimethylpentane, 2,2-dimethylpentane, 2,2,3-trimethylbutane, 2,2,3-trimethylbutane, cycloheptane, methylcyclohexane, ethylcyclopentane, n-octane, n-octane, 2-methylheptane, 3-methylheptane, 4-methylheptane, 2,2-dimethylhexane, 2,3-dimethylhexane, 2,4-dimethylhexane, 2,5-dimethylhexane, 3,3-dimethylhexane, 3,4-dimethylhexane, 3-ethylhexane, 2,2,3-trimethylpentane, 2,2,4-trimethylpentane (isooctane), 2,2,4-trimethylpentane, 2,3,3-trimethylpentane, 2,3,4-trimethylpentane, 2-methyl-3-ethylpentane, 3-methyl-3-ethylpentane, 2,2,3,3-tetramethylbutane, cyclooctane, methylcycloheptane, ethylcyclohexane, 1,1-dimethylcyclohexame, 1,2-dimethylcyclohexame, 1,3-dimethylcyclohexane, 1,4-dimethylcyclohexane, n-propylcyclopentane, n-nonane, nonane isomers represented by the molecular formula C₉H₂₀ (34 kinds), methylcyclooctane, ethylcycloheptane, n-propylcyclohexane, isopropylcyclohexane, 1,2,4-trimethylcyclohexane, 1-ethyl-3-methylcyclohexane, 1-ethyl-2-methylcyclohexane, n-butylcyclopentane, n-decane, decane isomers represented by the molecular formula C₁₀H₂₂ (74 kinds), cyclodecane, ethylcyclooctane, n-propylcycloheptane, n-butylcyclohexane, isobutylcyclohexane, tert-butylcyclohexane, 1-methyl-3-(n-propyl)cyclohexane, 1-methyl-2-(n-propyl)cyclohexane, 1,3-diethylcyclohexane, 1,2-diethylcyclohexane, 1,2,4,5-tetramethylcyclohexane, n-pentylcyclopentane, n-undecane, undecane isomers represented by the molecular formula C₁₁H₂₄ (158 kinds), methylcyclodecane, n-butylcycloheptane, n-pentylcyclohexane, n-dodecane, bicyclohexyl, 2,2,4,6,6-pentamethylheptane (isododecane), 2-methylundecane, dodecane isomers represented by the molecular formula C₁₂H₂₆, n-butylcyclooctane, n-pentylcycloheptane, n-hexylcyclohexane, n-tridecane, tridecane isomers represented by the molecular formula C₁₃H₂₈ (801 kinds), n-pentylcyclooctane, n-hexylcycloheptane, n-heptylcyclohexane, n-tetradecane, tetradecane isomers represented by the molecular formula C₁₄H₃₀ (1857 kinds), n-pentadecane, pentadecane isomers represented by the molecular formula C₁₅H₃₂ (4346 kinds), n-hexadecane, 2,2,4,4,6,8,8-heptamethylnonane (isohexadecane), hexadecane isomers represented by the molecular formula C₁₆H₃₄, n-heptadecane, heptadecane isomers represented by the molecular formula C₁₇H₃₆, n-octadecane, octadecane isomers represented by the molecular formula C₁₈H₃₈, n-nonadecane, 2,6,10,14-tetramethylpentadecane, nonadecane isomers represented by the molecular formula C₁₉H₄₀, n-eicosane, eicosane isomers represented by the molecular formula C₂₀H₄₂, and the like.

Among them, 2,2,4-trimethylpentane, cyclohexane, n-hexane, n-heptane, n-nonane, n-decane, and n-undecane are preferable. In particular, 2,2,4-trimethylpentane or n-heptane is preferable.

In addition, as an example of the saturated aliphatic hydrocarbon relevant to the present invention, kerosene is also enumerated. Kerosene may be the one which is generally commercially available.

For example, kerosene of this invention has been defined in "Act on the Quality Control of Gasoline and Other Fuels", Article 2, as "In this Act, "kerosene" means hydrocarbon oil which has a 95%-distillation temperature according to the testing method for distillation characteristics prescribed by Ordinance of the Ministry of Economy, Trade and Industry in a range not exceeding 270°C and not exceeding the temperature prescribed by Ordinance (volatile oils specified in section 2 are excluded)." And the standard thereof has been prescribed in Ordinance for Enforcement of the same Act, Article 27, as "Standard of kerosene in the Act, Article 17-9, section 1 prescribed by Ordinance of Ministry of Economy, Trade and Industry is as enumerated in the following items: 1. sulfur content is 0.008 % by mass or less; 2. flash point is 40°C or higher; 3. Saybolt color is +25 or more".

Specifically, kerosene is, for example, a mixture containing a plurality of saturated aliphatic hydrocarbons (having around 9 to 15 carbon atoms) described above as main components.

The first step in the purification method relevant to the present invention is "a step comprising contacting the compound represented by the general formula [1] or [2], a saturated aliphatic hydrocarbon having no substituent or kerosene relevant to the present invention with ozone gas". By this step, for example, impurities having a double bond is decomposed by contacting with ozone gas, and converted to a compound which can be easily separated and removed in the subsequent step. It should be noted that hereinafter, "a compound represented by the general formula [1] or [2], a saturated aliphatic hydrocarbon having no substituent or kerosene relevant to the present invention" to be applied in the first step is sometimes referred to as "raw material relevant to the present invention".

Ozone gas to be used in the first step may be a pure ozone gas, but when ozone gas is contacted with the raw material relevant to the present invention, it can be used by diluting with a gas such as nitrogen, argon, helium, carbon dioxide or air. When the term "ozone gas" is simply used in the present specification, both of a case of pure ozone gas and ozone gas diluted with, for example, a gas other than ozone as described above may be included.

Ozone gas can be obtained by feeding air, oxygen or oxygen-containing gas to an ozone generator. The ozone generator is not particularly limited, and the one having preferable specifications (size and the like) corresponding to the circumstance where the first step is carried out may be appropriately selected and used, and various types of commercially available ozone generators may be appropriately used.

Amount of ozone gas to be fed to the raw material relevant to the present invention is desirably an amount exceeding the amount of ozone gas which is required for eliminating impurities contained in the raw material relevant to the present invention to such amount that can be removed completely in the subsequent second step. That is, the desirable amount is 1 to 5 times, and preferably 1.5 to 3 times of the amount of ozone gas which is required for eliminating impurities contained in the raw material relevant to the present invention to such amount that can be removed completely in the subsequent second step.

It can be judged, for example, by analyzing ozone concentrations in the ozone gas and the exhaust gas discharged after contacting the raw material relevant to the present invention with ozone gas, whether impurities contained in the raw material relevant to the present invention has been eliminated to such amount that can be removed completely in the subsequent second step. Specifically, when ozone concentration in the exhaust gas becomes 80 % or more, and preferably 90 % or more of ozone concentration in the feed gas, it can be judged that "impurities have been eliminated to such amount that can be removed completely in the subsequent second step". Analysis of ozone concentration in the exhaust gas may be performed by common procedures such as iodine titration method, UV absorption method, chemiluminescence method, and the like. In practice, the analysis may be carried out using a continuous analyzer or the like utilizing these methods.

In addition, for example, a part of the raw material relevant to the present invention is collected as a sample at regular time intervals while the raw material relevant to the present invention is contacted with ozone gas, and when prescribed fluorescence intensity or absorbance at prescribed wavelength of the raw material relevant to the present invention becomes a given value or lower, it can be also judged that "impurities have been eliminated to such amount that can be removed completely in the subsequent second step".

An amount of ozone gas to be fed to the raw material relevant to the present invention varies depending on the raw material relevant to the present invention, but the amount is, when shown in amount of ozone gas aerated (as O₃) to the raw material relevant to the present invention, roughly around 0.001 g to 1.0 g, and preferably 0.002 g to 0.20 g of ozone to 1 L of the raw material, or around 0.2 g to 200 g, and preferably 0.4 g to 40 g of ozone to 200 L of the raw material relevant to the present invention.

Ozone concentration in ozone gas is 0.01 to 5.0 % by volume (about 0.2 to 107.1 g/Nm³: ozone amount (g) in 1 m³ of ozone gas), and preferably 0.05 to 2.0 % by volume (about 1.1 to 42.8 g/Nm³). Too low ozone concentration is not preferable because the time required for contact of the raw material relevant to the present invention and ozone gas becomes prolonged, as well as reaction vessel becomes large, and the like.

Time for feeding ozone gas may be "a time required for eliminating impurities contained in the raw material relevant to the present invention to such amount that can be removed completely in the subsequent second step" as described above or longer. Alternatively, the time may be "a time required for prescribed fluorescence intensity or absorbance at prescribed wavelength of the raw material relevant to the present invention to become a given value or lower" as described above or longer.

Temperature at which the raw material relevant to the present invention is contacted with ozone gas is around -40 to 80°C, preferably around 5 to 60°C, and more preferably around 10 to 40°C. Too low temperature has such problems that reaction rate becomes slow, and trace component deposits, or the like. Also, too high temperature facilitates the reaction excessively, and sometimes lowers yield of the raw material relevant to the present invention. Therefore, around room temperature (20°C) is sufficient.

Pressure for contacting the raw material relevant to the present invention with ozone gas may be any one of reduced pressure, ordinary pressure or under pressure, and may be appropriately determined correspondingly to feed condition of ozone gas and the like.

Method for contacting the raw material relevant to the present invention with ozone gas may be either batch style (batch type) or continuous style (flow-through type). In view of simple and convenient operation, batch style is preferable.

Each method is explained, for example, as follows.

### (1) Batch style (batch type)

For example, reaction may be carried out for a prescribed period of time by continuously aerating a prescribed concentration of ozone gas into a reaction vessel containing a prescribed amount of the raw material relevant to the present invention, while controlling the temperature and the pressure if necessary. In addition, in order to improve contact of the raw material relevant to the present invention with ozone gas, the step is desirably carried out in a reaction vessel with a stirring device. Furthermore, in order to improve gas-liquid contact, ozone gas is preferably fed into the raw material relevant to the present invention from nozzles provided at one or more positions in a form of small bubbles (so-called bubbling).

In the batch style, as for contact time of the raw material relevant to the present invention with ozone gas, preferably the above-described amount of ozone gas is fed over 1 to 600 minutes, preferably 5 to 300 minutes, and more preferably 10 to 120 minutes. Too short feed time may cause such problems that reaction between ozone and impurities becomes insufficient, or the raw material relevant to the present invention is blown due to too great gas flow rate or the like to increase loss. In addition, too long feed time has no merit in productivity, as well as causes such problem that oxidation reaction is facilitated excessively.

Exhaust gas contacted with the raw material relevant to the present invention after the ozone gas treatment may be discharged by appropriately passing through an ozone decomposition layer such as activated charcoal.

### (2) Continuous style (flow-through type)

For example, there is included a method where the raw material relevant to the present invention is continuously added to ozone gas which is continuously fed, contacted and treated for a prescribed period of time, and then the raw material relevant to the present invention is continuously taken out from the system, and the like.

It should be noted that, in this specification, "contacting" the raw material relevant to the present invention with ozone gas is sometimes referred to as "treating" the raw material relevant to the present invention with ozone gas.

The second step in the purification method relevant to the present invention is a step comprising contacting the raw material relevant to the present invention passed through the first step (hereinafter, sometimes referred to as "semi-purified product relevant to the present invention", or simply "semi-purified product") with zeolite or a basic adsorbent. In the step, one kind of zeolite or basic adsorbent may be used, or plural kinds of zeolite, plural kinds of basic adsorbents, or one or more kinds of zeolite and one or more kinds of basic adsorbents may be used.

In view of easy and simple handling, use of zeolite is more preferable.

Zeolite relevant to the present invention to be used in the second step may be either natural zeolite or synthetic zeolite. It includes, for example, X-type zeolite, A-type zeolite, Y-type zeolite, and the like.

Since zeolite has various types of commercially available products, those products may be used. Commercially available zeolite to be used in the second step includes synthetic zeolite F-9 (X-type zeolite), synthetic zeolite A-3 (A-type zeolite), synthetic zeolite A-4 (A-type zeolite), synthetic zeolite HS-320 (Y-type zeolite), molecular sieves 5A (A-type zeolite) (all above are supplied by Wako Pure Chemical Industries, Ltd.), and the like. Among them, synthetic zeolite F-9 is preferable.

Shape of zeolite to be used in the present invention may be any one of powdery, finely granular, and granular, and may be appropriately selected correspondingly to an amount of the semi-purified material to be treated, scale of treatment, and the like.

The basic adsorbent relevant to the present invention to be used in the second step includes anion-exchange resin; hydroxide, carbonate salt or bicarbonate salt of alkali metal; oxide, hydroxide or carbonate of alkaline earth metal; solid base of a mixture of different kinds of metal oxides; substance where alkali metal or alkaline earth metal compound is supported on a carrier; and the like.

Since these basic adsorbents also have various kinds of commercially available products, those products may be used. Commercially available basic adsorbent includes, for example, basic activated alumina which is also used for affinity chromatography, and the like.

Method for contacting the semi-purified product relevant to the present invention with zeolite or basic adsorbent may be either batch style (batch type) or continuous style (flow-through type). For industrial use, continuous style is preferable.

Each method is explained as follows.

### (1) Batch style (batch type)

The semi-purified product obtained in the first step is mixed with the zeolite or the basic adsorbent relevant to the present invention, and the mixture is subjected to shaking treatment. By this operation, only impurities in the semi-purified product are adsorbed on the zeolite or the basic adsorbent. Subsequently, by separating the obtained purified product and the zeolite or the basic adsorbent, only impurities can be removed from the purified product, and the purified product can be obtained. In addition, for separating the purified product and the zeolite or the basic adsorbent, for example, an appropriate technique such as filtration, decantation, or the like may be used.

In the batch style, the semi-purified product may be treated with one kind of zeolite or basic adsorbent, or may be treated with a mixture of plural kinds of zeolites, a mixture of plural kinds of basic adsorbents, or a mixture of one or more kinds of zeolites and one or more kinds of basic adsorbents.

As for amount of the zeolite or the basic adsorbent relevant to the present invention in the case of batch style, greater amount can remove impurities in the semi-purified product more sufficiently, but too much amount is fruitless and not economic. Therefore, in view of an economic amount, for example, ratio of amounts of the zeolite or the basic adsorbent relevant to the present invention to be used to semi-purified product may be around 1 : 5 to 1 : 10000, and preferably 1 : 10 to 1 : 1000. It should be noted that the ratio may be any one of weight ratio (W/W), volume ratio (V/V), or volume/weight ratio (V/W). For example, amount of zeolite to be used may be 1 to 5 kg relative to 200 L of the raw material.

In addition, time for contacting the semi-purified product with the zeolite or the basic adsorbent relevant to the present invention may be a time which is sufficient for impurities in the semi-purified product to be adsorbed on the zeolite or basic adsorbent, and is, for example, 0.1 to 1000 minutes, and preferably 1 to 300 minutes.

### (2) Continuous style (flow-through type)

This style includes a means (column method) where the semi-purified product obtained in the first step is applied to a column (packed tower) packed with the zeolite or the basic adsorbent relevant to the present invention, and the like. In addition, in the column method, general liquid chromatography method is enough. This method is industrially advantageous from the viewpoint that a large amount of sample can be treated at a time.

In the continuous style, a column packed with one kind of the zeolite or the basic adsorbent may be used, or any one of a column where plural kinds of zeolites are stacked, a column where plural kinds of basic adsorbents are stacked, or a column where one or more kinds of zeolites and one or more kinds of basic adsorbents are stacked can be used.

The column method may be carried out, for example, as follows.

Firstly, the semi-purified product obtained in the first step is flowed through a column (packed column) packed with the zeolite or the basic adsorbent relevant to the present invention. By this operation, only impurities such as UV absorbing substance, fluorescent substance, and sulfur compound or oxides thereof are adsorbed on the zeolite or the adsorbent. And the purified product not containing impurities is eluted from the column. This eluate may be recovered.

Size of the column to be used is not particularly limited, and may be appropriately selected correspondingly to amount of semi-purified product to be treated. The column which is commonly used for cleaning up treatment for a sample for dioxins analysis or a sample for PCBs analysis may be used. For example, a column having an inner diameter (ϕ) of 5 to 1000 mm, and preferably 10 to 600 mm, and length of 30 to 5000 mm, and preferably 100 to 3000 mm is enumerated.

The packed column to be used in the purification method of the present invention can be obtained by packing the zeolite or the basic adsorbent relevant to the present invention into a column according to the common method such as wet process or dry process packing method.

Amount of the zeolite or the basic adsorbent relevant to the present invention to be packed to column is same as in the case of batch style of treatment, and ratio of amounts of the zeolite or the basic adsorbent relevant to the present invention and the semi-purified product relevant to the present invention to be used may be around 1 : 5 to 1 : 10000, and preferably 1 : 10 to 1 : 1000. It should be noted that the ratio may be any one of weight ratio (W/W), volume ratio (V/V), or volume/weight ratio (V/W). For example, amount of the zeolite to be used may be 1 to 5 Kg to 200 L of the raw material.

Flow rate of the eluate in space velocity (SV) is appropriately selected from a range of 0.01 to 1000 (H⁻¹) [SV = 1 (H⁻¹): a rate required for treating 1 L of solvent with 1 L of adsorbent per 1 hour], and preferably 0.1 to 100 (H⁻¹), but not limited thereto.

Temperature at which the semi-purified product is contacted with the zeolite or the basic adsorbent relevant to the present invention in the second step of the present invention is from -40 to 80°C, preferably 5 to 60°C, and more preferably from 10 to 40°C.

It should be noted that, in this specification, "contacting" the semi-purified product relevant to the present invention with the zeolite or the basic adsorbent relevant to the present invention is sometimes referred to as "treating" the semi-purified product relevant to the present invention with the zeolite or the basic adsorbent relevant to the present invention.

The eluate obtained in the second step is a purified product of the raw material relevant to the present invention, and does not require further treatment such as distillation.

For example, since 1,2,4-trichlorobenzene as a raw material has a high fluorescence at exciting wavelength 365 nm and hence is not suitable to be used as a mobile phase for liquid chromatography, the raw material has been conventionally purified through zeolite treatment. In this conventional method, such a large amount as about 40 to 50 kg of zeolite to 200 L of the raw material was needed to be used. Also, there has been a problem that sometimes 1,2,4-trichlorobenzene contains around 0.1 to 0.5 ppm of PCB. Therefore, when 1,2,4-trichlorobenzene is purified by the conventional purification method using zeolite, the zeolite is contaminated with PCB, and a need has been occurred that a large amount of the zeolite used for purification has to be treated as a PCB waste.

According to the purification method of the present invention, by contacting the raw material with ozone gas before the zeolite treatment, it has become possible to reduce amount of the zeolite required for the subsequent zeolite treatment to much less amount. In addition, the purification method of the present invention can be satisfied only by substantially subjecting the raw material relevant to the present invention to the treatment by ozone gas and the treatment by the zeolite or the basic adsorbent relevant to the present invention, and is not required to carry out purification treatment such as distillation additionally. A purified product suitable to use as a mobile phase for liquid chromatography can be obtained only by the purification method of the present invention.

Hereinafter, the present invention will be explained more in detail by means of Examples, however, the present invention is not limited by these Examples.

### EXAMPLES

### Experimental Example 1: Purification of 1,2,4-trichlorobenzene using an adsorbent

By using 1,2,4-trichlorobenzene as a raw material, and contacting the raw material with various kinds of adsorbents by batch-wise treatment, removal effect of each adsorbent on removal of fluorescent substance from 1,2,4-trichlorobenzene was studied.

### (1) Raw material: 1,2,4-trichlorobenzene (raw material for industrial use)

### (2) Adsorbent

* Synthetic zeolite F-9 (supplied by Wako Pure Chemical Industries, Ltd.);
* Activated charcoal (granular) (Wako special grade, produced by Wako Pure Chemical Industries, Ltd.);
* Activated charcoal (for column chromatography use, produced by Wako Pure Chemical Industries, Ltd.);
* Wako Gel C-200 (for column chromatography use, produced by Wako Pure Chemical Industries, Ltd.);
* Activated alumina (for column chromatography use, produced by Wako Pure Chemical Industries, Ltd.);
* Molecular Sieves 5A (supplied by Wako Pure Chemical Industries, Ltd.).

### (3) Treatment with adsorbent

Raw material (500 mL) was poured into a 100 mL Erlenmeyer flask, any one of the above-described adsorbents (1 g) was added thereto, and the mixture was stirred for 5 hours. After that, the treated liquid was filtered through a membrane filter (0.45 µm).

### (4) Measurement

Using a fluorescence spectrophotometer (manufactured by Hitachi High-Technologies Corp., F-4500), fluorescence intensities of the filtrates obtained in the above item (3) were measured at exciting wavelength 365 nm and fluorescence wavelength 450 to 700 nm.

### (5) Results

The obtained results are shown in Table 1 below. The value in Table 1 is a value converted to quinine sulfate of fluorescence intensities at exciting wavelength 365 nm and fluorescence wavelength 450 to 700 nm [a value converted to relative fluorescence intensity (R.F.I) based on a value of fluorescence peak value of 10 µg/L quinine sulfate solution (acidified by 0.1 N sulfuric acid) at exciting wavelength 365 nm / fluorescence wavelength around 450 nm being 10 Q.S.U. (quinine sulfate unit)] of raw materials treated with each adsorbent.

**[Table 1]**

| Fluorescence wavelength | Standard value for HPLC | Raw material | Adsorbent | | | | | |
|---|---|---|---|---|---|---|---|---|
| | | | Synthetic zeolite F-9 | Activated charcoal (granular) | Activated charcoal (for chromatography) | Wako gel C-200 | Activated alumina | Molecularsieves 5A |
| 450 - 490 nm | 5 or less | 22.1 | 12.3 | 31.4 | 60.7 | 19.7 | 21.6 | 17.5 |
| 500 - 540 nm | 3 or less | 7.2 | 3.5 | 12.4 | 19 | 5.8 | 6.6 | 5.5 |
| 550 - 590 nm | 3 or less | 2.2 | 1 | 3.9 | 5.1 | 1.7 | 1.9 | 1.6 |
| 600 - 700 nm | 3 or less | 0.5 | 0.3 | 0.7 | 0.8 | 0.4 | 0.4 | 0.4 |

As obvious from Table 1, a fluorescent substance was present in the raw material before treating with the adsorbents, and fluorescent intensities thereof were higher values than the standard value for fluorescent substance required for high performance liquid chromatography (HPLC) use.

In the treatment of the raw material with various kinds of adsorbents, amount of the fluorescent substance was most remarkably reduced when the raw material is treated with synthetic zeolite F-9. However, such treated raw material was still insufficient as a standard product for HPLC use. Considering decrease in fluorescence intensity at any fluorescence wavelength compared with that before the treatment, it can be understood that the synthetic zeolite F-9 has a high removal effect for fluorescent substance from 1,2,4-trichlorobenzene.

### Example 1

A purification method was studied, where ozone gas treatment was added to the treatment method using the synthetic zeolite F-9 which was confirmed to have a high removal effect for fluorescent substance in Experimental Example 1.

### (1) Raw material: 1,2,4-trichlorobenzene (raw material for industrial use)

### (2) Treatment with ozone gas

### 1) Ozone generator

Ozone generator for experimental use: Kofloc PZ-1A (manufactured by Kofloc Inc.)

### 2) Treatment method

The raw material (100 mL) was poured into a 200 mL glass container provided with an ozone gas aeration tube connected to the ozone generator and an exhaust gas discharging tube. Ozone gas was bubbled into the raw material using the ozone generator under the following conditions, while the raw material was stirred.

Ozone gas aeration conditions (ordinary pressure)
amount of ozone gas to be generated: 0.3 g/hr;
flow rate of gas: 1 L/min;
treatment temperature: 20°C;
treatment time: 10 min (amount of ozone gas aerated: 0.05 g / 100 mL of raw material).

Ozone concentration in the ozone gas when aerated under the above gas aeration conditions was about 0.2335 vol % (about 5 g/Nm³).
A part of the semi-purified product after the ozone gas treatment was collected as a sample.

### (3) Treatment with adsorbent

The ozone-gas-treated semi-purified product was applied to a column where synthetic zeolite F-9 (10 g) was packed in a ϕ 2 cm glass column, and the semi-purified product was flowed through at a 1.2 mL/min of treating velocity (space velocity ((SV) = 5.0 (H⁻¹)). After cutting off the initial eluate (5 mL), the eluate was recovered.

### (4) Measurement

For the recovered eluate and the ozone-gas-treated semi-purified product collected as a sample, fluorescence intensity at exciting wavelength 365 nm and fluorescence wavelength 450 to 700 nm was measured by the same equipment and method as in Experimental Example 1-(4).

In addition, UV absorbance at wavelength 200 to 400 nm was also measured using a spectrophotometer (manufactured by Hitachi High-Technologies Corp., U-3310). It should be noted that the measurement value of UV absorbance was a value measured according to JIS K 0115 where absorbance at each wavelength was measured using an absorption cell (10 mm) made of quartz glass and distilled water as a reference liquid.

Aside from this, for the eluate obtained by treating only with the above-described zeolite without treating with ozone gas, fluorescence intensity was measured in the same manner.

### (5) Results

The obtained results are shown in Table 2. In Table 2, the absorbance at 380 to 400 nm shows a maximum value of the absorbance at 380 to 400 nm. And also, the fluorescence intensity shows a maximum value of the fluorescence intensity by a quinine sulfate equivalent.

**[Table 2]**

| Inspection item | | Raw material | Zeolite treatment only | Ozone gas treatment, then zeolite treatment |
|---|---|---|---|---|
| Absorbance | 310 nm | 0.446 | 0.319 | 0.285 |
| | 320 nm | 0.194 | 0.063 | 0.064 |
| | 350 nm | 0.194 | 0.043 | 0.046 |
| | 370 nm | 0.127 | 0.016 | 0.016 |
| | 380 - 400 nm | 0.090 | 0.004 | 0.004 |
| Fluorescence inetnsity | 450 - 490 nm | 20.0 | 8.4 | 1.7 |
| | 500 - 540 nm | 7.2 | 3.4 | 0.6 |
| | 550 - 590 nm | 2.2 | 1.2 | 0.3 |
| | 600 - 700 nm | 0.6 | 0.4 | 0.2 |

As obvious from Table 2, it can be understood that by treating the raw material with ozone gas before treating with adsorbent, removal effect for fluorescent substance can be further improved compared with that of only treatment with adsorbent.

### Example 2

Optimum conditions of the ozone gas treatment and the adsorbent treatment were studied.

### (1) Raw material: 1,2,4-trichlorobenzene (raw material for industrial use).

### (2) Treatment with ozone gas

1) Ozone generator: same as the one used in Example 1.
2) Treatment method
The raw material (650 mL) was poured into a 1 L glass container provided with an ozone gas aeration tube connected to ozone generator and an exhaust gas discharging tube, and ozone gas was bubbled into the raw material using the ozone generator under each condition (ordinary pressure) described in the Table 3 below, while stirring the raw material.

It should be noted that in Table 3, "amount of ozone gas aerated (value converted to g/l L)" shows an amount of ozone (as O₃) aerated into the raw material (650 mL) in each experiment as a value converted to an amount of ozone aerated (as O₃) to 1 L of the raw material, and "amount of ozone gas aerated (value converted to g / 200 L)" shows a value converted as amount of ozone aerated (as O₃) to 200 L of the raw material.

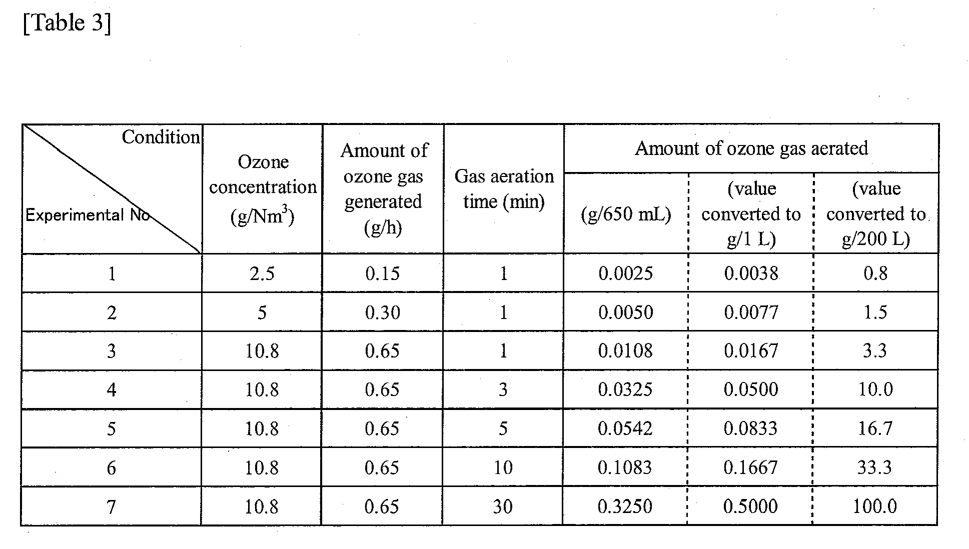

Apart of the semi-purified product after the ozone gas treatment was collected as a sample.

### (3) Treatment with adsorbent

The total amount of each ozone-gas-treated semi-purified product was applied to a column where synthetic zeolite F-9 (10 g) was packed in a ϕ 2 cm glass column, and the semi-purified product was flowed through at a 1.2 mL/min of treating velocity (SV = 5.0 (H⁻¹)). After cutting off the initial eluate (5 mL), the eluate was fractionated in 200 mL × 2 fractions, and then the third fraction (only this fraction: 230 mL) was fractionated.

### (4) Measurement

For the recovered eluate in each fraction and the ozone-gas-treated semi-purified product corrected as a sample, fluorescence intensity at exciting wavelength 365 nm and fluorescence wavelength 450 to 700 nm was measured by the same equipment and method as in Experimental Example 1-(4). And also, UV absorbance at wavelength 350 nm was measured using a spectrophotometer.

### (5) Results

The obtained results are shown in Table 4. In Table 4, fluorescence intensity shows a maximum value of the fluorescence intensity under the above-described measurement conditions as a quinine sulfate equivalent. It should be noted that in any case of Experimental Nos. 1 to 5, the maximum value of fluorescence intensity appeared at fluorescence wavelength 450 to 490 nm. Further, in any case of Experimental Nos. 1 to 5, fluorescence intensities were less than 3 in the range of fluorescence wavelength 500 to 700 nm in all cases.

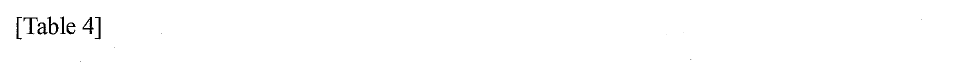

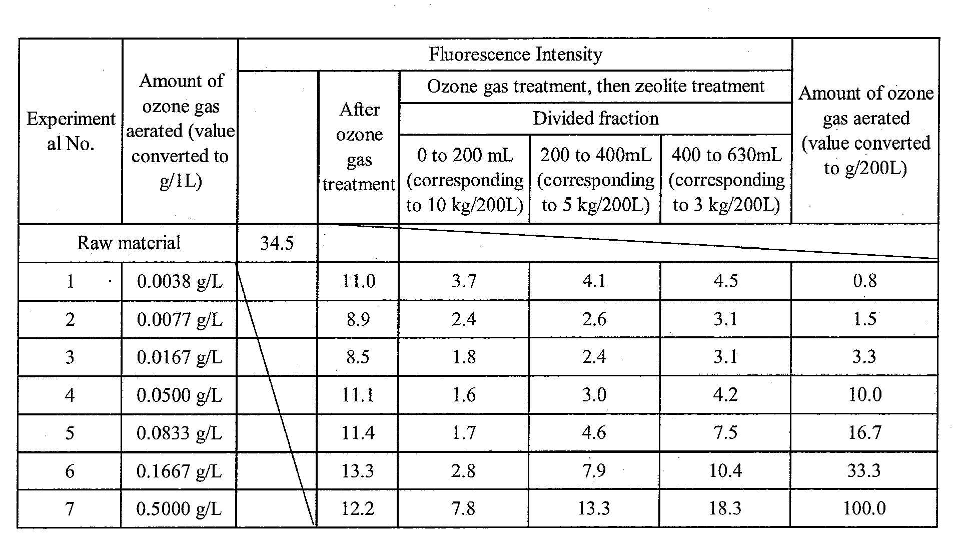

In addition, measurement results of absorbance are shown in Table 5. In Table 5, absorbance is expressed by "○" when absorbance is conformable to the standard of HPLC use (when UV absorbance at 350 nm is 0.05 or less), and by "×" when absorbance is not conformable thereto.

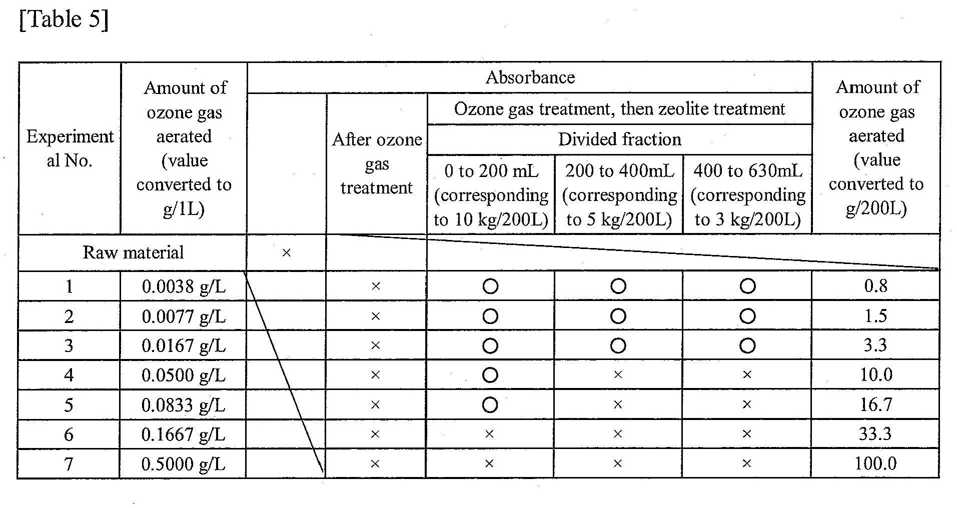

As obvious from the results of Table 4 and Table 5, it can be understood that by treating the raw material with ozone gas under the conditions in Experimental Nos. 1 to 3, and subsequently treating with an adsorbent, a purified product conformable with the standard product for HPLC use in both of fluorescence intensity and absorbance could be obtained.

In addition, in the zeolite treatment in Example 2, the eluate flowed through the zeolite column was collected by dividing into 3 times. Since 200 mL of the first sampling liquid was treated with 10 g of zeolite, this corresponds to the case where 200 L of the raw material is treated with about 10 kg of zeolite, when converted to the case where 200 L of the raw material is treated in a real production scale. Similarly, the second sampling liquid corresponds to the case where 400 mL of the raw material was treated with 10 g of zeolite, and this corresponds to the case where 200 L of the raw material is treated with about 5 kg of zeolite, when converted to the real production scale. The third sampling liquid corresponds to the case where 630 mL of the raw material was treated with 10 g of zeolite, and this corresponds to the case where 200 L of the raw material is treated with about 3 kg of zeolite, when converted to the real production scale. Therefore, in comparison of the measurement results of fluorescence intensity and absorbance in Experimental Nos. 1 to 3 in Table 4 and Table 5 with an amount of ozone gas aerated (value converted to g/200 L), it can be understood that when 200 L of the raw material is treated with about 1 to 3 g/200 L of ozone, purification becomes possible by using about 3 kg/200 L (raw material) of zeolite in the subsequent zeolite treatment. By comparing with the existing amount of zeolite to be used of 40 to 50 kg/200 L (raw material), it was indicated that amount of zeolite to be used could be drastically reduced by treating the raw material with ozone gas and subsequently treating with zeolite according to the method of the present invention.

It should be noted that, when ozone gas was excessively aerated, the raw material became colored, and both of absorbance and fluorescence intensity were not improved despite of the zeolite treatment.

### Example 3

The purification method relevant to the present invention was executed in the real production scale. (1) Raw material: 1,2,4-trichlorobenzene (raw material for industrial use).

### (2) Treatment with ozone gas

1) Ozone generator: same to the one used in Example 1.
2) Treatment method:
   The raw material (200 L) was poured into a drum provided with an ozone gas aeration nozzle connected to the ozone generator and an exhaust gas discharging tube. Ozone gas was bubbled into the raw material in fine bubbles through the ozone gas aeration bubble provided in the bottom section in the drum under the following conditions, while the raw material was stirred.

Ozone gas aeration conditions (ordinary pressure)
amount of ozone gas to be generated: 1 g/hr;
flow rate of gas: about 2 L/min;
treatment temperature: 20°C.
Ozone concentration in the ozone gas when aerated under the above gas aeration conditions was about 0.38761 vol % (about 8.3 g/Nm³).

Aeration of ozone gas was stopped every 30 minutes, the raw material was collected as a sample, and fluorescence intensity at excitation wavelength 365 nm and fluorescence wavelength 450 to 700 nm was measured by the same equipment and method as in Experimental Example 1-(4). When fluorescence intensity became 10 or less (converted to quinine sulfate), bubbling was stopped.

A part of the ozone-gas-treated semi-purified product was collected as a sample.

### (3) Treatment with adsorbent

The total amount of each ozone-gas-treated semi-purified product was applied to a column where synthetic zeolite F-9 (5 kg) was packed in a ϕ 30 cm glass column, and the semi-purified product was flowed through at a 33 L/hr of treating velocity (SV = 4.6 (H⁻¹)). After cutting off the initial eluate (3 L), the eluate was recovered.

### (4) Measurement

For the recovered eluate and the ozone-gas-treated semi-purified product collected as a sample, fluorescence intensities at exciting wavelength 254 nm and fluorescence wavelength 450 to 700 nm, and at exciting wavelength 365 nm and fluorescence wavelength 450 to 700 nm were measured by the same equipment and method as in Experimental Example 1-(4). Further, UV absorbance at wavelength 200 to 400 nm was also measured using a spectrophotometer.

### (5) Results

The results are shown in Table 6 below. In Table 6, the absorbance at 380 to 400 nm shows the maximum value of the absorbance at 380 to 400 nm. Further, the fluorescence intensity shows the maximum value of the fluorescence intensities under the above-described conditions as a quinine sulfate equivalent.

It should be noted that the maximum value of the fluorescence intensity appeared at fluorescence wavelength 450 to 490 nm. Further, within a range of fluorescence wavelength 500 to 700 nm, all of the fluorescence intensities were less than 3.

**[Table 6]**

| Inspection item | | Raw material | After ozone gas treatment | Ozone gas treatment, then zeolite treatment |
|---|---|---|---|---|
| Absorbance | 310 nm | 0.342 | 0.402 | 0.324 |
| | 320nm | 0.105 | 0.156 | 0.042 |
| | 350 nm | 0.086 | 0.112 | 0.041 |
| | 370 nm | 0.053 | 0.069 | 0.017 |
| | 380 - 400 nm | 0.037 | 0.048 | 0.005 |
| Fluorescence intensity | Ex: 254 nm (max) | - | - | 2.2 |
| | Ex: 365 nm (max) | 30.4 | 8.1 | 3.6 |

As obvious from the results of Table 6, it can be understood that a purified product suitable for use as a mobile phase for HPLC can be obtained by treating the raw material with ozone gas and subsequently treating with zeolite, even further purification treatment such as distillation is not carried out. In addition, by the conventional method where the zeolite treatment is carried out without carrying out the ozone gas treatment, about 40 to 50 kg of zeolite for 200 L of raw material had been used. On the other hand, it can be also understood that by the purification method relevant to the present invention, the purification treatment can be performed with a far smaller amount (about 5 kg) of zeolite to be used compared with that of the conventional method.

### Example 4

### (1) Raw material: 2,2,4-trimethylpentane (raw material for industrial use).

### (2) Treatment with ozone gas

1) Ozone generator: same to the one used in Example 1.
2) Treatment method:
The raw material (400 mL) was poured into a 1 L glass container provided with an ozone gas aeration tube connected to the ozone generator and an exhaust gas discharging tube. Ozone gas was bubbled into the raw material under the following conditions, while the material was stirred.

Ozone gas aeration conditions (ordinary pressure)
amount of ozone gas to be generated: 0.4 g/hr;
flow rate of gas: about 1 L/min;
treatment temperature: about 20°C (room temperature);
treatment time:
(a) 1 minute;
(b) 3 minutes;
(c) 5 minutes.
Ozone concentration in the ozone gas when aerated under the above gas aeration conditions was about 0.31289 vol % (about 6.7 g/Nm³).

It should be noted that total amount of ozone gas aerated under the condition of treatment time (a) 1 minute is 0.4 g × (1/60 min) = 0.007 g/400 mL raw material (= 0.017 g/L raw material). Similarly, total amount of ozone gas aerated under the condition of treatment time (b) 3 minutes is 0.4 g × (3/60 min) = 0.02 g/400 mL raw material (= 0.05 g/L raw material). Total amount of ozone gas aerated under the condition of treatment time (c) 5 minutes is 0.4 g × (5/60 min) = 0.033 g/400 mL raw material (= 0.083 g/L raw material).

A part of the semi-purified product treated with ozone gas for each treatment time was collected as a sample.

### (3) Treatment with adsorbent

The ozone-gas-treated semi-purified product (350 mL) was applied to a column where synthetic zeolite F-9 (10 g) was packed in a ϕ 2 cm glass column, and the semi-purified product was flowed through at a 1 mL/min of treating velocity (SV = 4.2 (H⁻¹)). After cutting off the initial eluate (10 mL), the eluate was recovered.

### (4) Measurement

For the recovered eluate and the ozone-gas-treated semi-purified product collected as a sample, fluorescence intensities at exciting wavelength 254 nm and fluorescence wavelength 450 to 700 nm, and at exciting wavelength 365 nm and fluorescence wavelength 450 to 700 nm were measured by the same equipment and method as in Experimental Example 1-(4). Further, UV absorbance at wavelength 200 to 400 nm was also measured using a spectrophotometer.

### (5) Results

The results are shown in Table 7 below. In Table 7, the absorbance at 254 to 400 nm shows the maximum value of the absorbance at 254 to 400 nm. Further, the fluorescence intensity shows the maximum value of the fluorescence intensity under the above-described measurement conditions as a quinine sulfate equivalent.

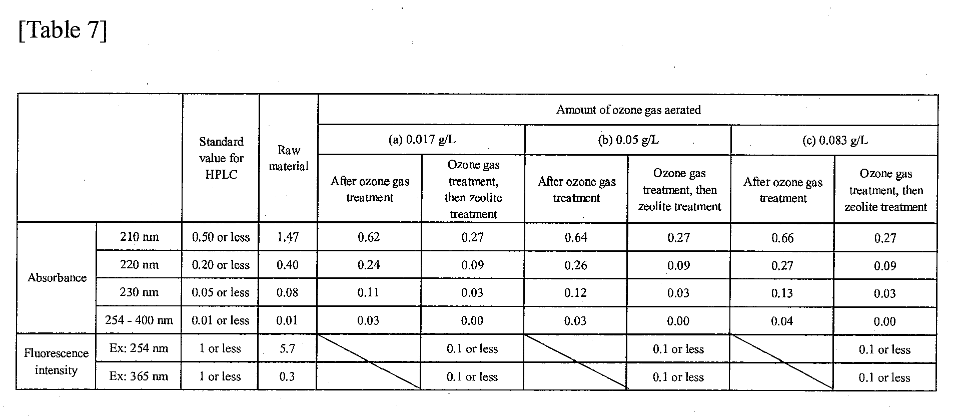

As obvious from the results of Table 7, it can be understood that 2,2,4-trimethylpentane suitable for use as a mobile phase for HPLC can be obtained by executing the purification method of the present invention.

### Example 5

### (1) Raw material: 1,2,4-trichlorobenzene (raw material for industrial use).

### (2) Treatment with ozone gas

1) Ozone generator: same as the one used in Example 1.
2) Treatment method
The raw material (650 mL) was poured into a 1 L glass container provided with an ozone gas aeration tube connected to the ozone generator and an exhaust gas discharging tube. Ozone gas was bubbled into the raw material using the ozone generator under the following conditions, while the raw material was stirred.

Ozone gas aeration conditions (ordinary pressure)
amount of ozone gas to be generated: 0.3 g/hr;
flow rate of gas: 1 L/min;
treatment temperature: 20°C;
treatment time: 2 minutes (amount of ozone gas aerated: 0.01 g/650 mL raw material)..

Ozone concentration in the ozone gas when aerated under the above gas aeration conditions was about 0.2335 vol % (about 5 g/Nm³).
A part of the ozone-gas-treated semi-purified product was collected as a sample.

### (3) Treatment with adsorbent

### 1) Adsorbent

* synthetic zeolite F-9 (supplied by Wako Pure Chemical Industries, Ltd.);
* synthetic zeolite HS-320 (Y-type zeolite, produced by Wako Pure Chemical Industries, Ltd.);
* basic activated alumina (for column chromatography use, produced by Wako Pure Chemical Industries, Ltd.).

### 2) Treatment method

The ozone-gas-treated semi-purified product (200 mL) was applied to a column where any one of the adsorbents described in the above item 1) (10 g) was packed in a ϕ 2 cm glass column, and the semi-purified product was flowed through at a 1.2 mL/min of treating velocity (space velocity SV = 5.0 (H⁻¹)). After cutting off the initial eluate (5 mL), the eluate was recovered.

### (4) Measurement

For the recovered eluate and the ozone-gas-treated semi-purified product collected as a sample, fluorescence intensity at exciting wavelength 365 nm and fluorescence wavelength 450 to 700 nm was measured by the same equipment and method as in Experimental Example 1-(4). Further, UV absorbance at wavelength 200 to 400 nm was also measured using a spectrophotometer.

### (5) Results

The results are shown in Table 8. In Table 8, the absorbance at 380 to 400 nm shows the maximum value of the absorbance at 380 to 400 nm. Further, the fluorescence intensity shows the maximum value of the fluorescence intensity at each fluorescence wavelength as a quinine sulfate equivalent.

**[Table 8]**

| Inspection item | | Raw material | After ozone gas treatment | Ozone gas treatment, then adsorbent treatment | | |
|---|---|---|---|---|---|---|
| | | | | Synthetic zeolite F-9 | Synthetic zeolite HS-320 | Basic activated alumina |
| Absorbance | 310 nm | 0.313 | 0.457 | 0.261 | 0.277 | 0.308 |
| | 320 nm | 0.071 | 0.183 | 0.041 | 0.052 | 0.078 |
| | 350 nm | 0.054 | 0.085 | 0.039 | 0.041 | 0.046 |
| | 370 nm | 0.021 | 0.033 | 0.014 | 0.015 | 0.016 |
| | 380 - 400 nm | 0.008 | 0.015 | 0.003 | 0.003 | 0.004 |
| Fluorescence intensity | Ex: 365 nm (max) | 34.5 | 9.9 | 1.7 | 3.0 | 2.6 |

As obvious from the results of Table 8, by executing the purification method of the present invention, absorbance and fluorescence intensity of the raw material could be reduced, even when Y-type zeolite (synthetic zeolite HS-320) or a basic adsorbent (basic activated alumina) was used. In addition, raw material could be purified to such a level that absorption and fluorescence intensity of the raw material was suitable for use as a mobile phase for HPLC.

In particular, it can be understood that the highest purification effect can be obtained when synthetic zeolite F-9 is used as an adsorbent.

### Example 6

### (1) Raw material: n-heptane (raw material for industrial use).

### (2) Treatment with ozone gas

1) Ozone generator: same to the one used in Example 1.
2) Treatment method:
The raw material (200 mL) was poured into a 1 L glass container provided with an ozone gas aeration tube connected to the ozone generator and an exhaust gas discharging tube. Ozone gas was bubbled into the raw material under the following conditions using the ozone gas generator, while the material was stirred.

. Ozone gas aeration conditions (ordinary pressure)
amount of ozone gas to be generated: 0.3 g/hr;
flow rate of gas: 1 L/min;
treatment temperature: 20°C;
treatment time: 2 minutes (amount of ozone gas aerated: 0.01 g/200 mL raw material).
Ozone concentration in the ozone gas when aerated under the above-described conditions was about 0.2335 vol % (about 5 g/Nm³).
A part of the ozone-gas-treated semi-purified product was collected as a sample.

### (3) Treatment with adsorbent

### 1) Adsorbent

* synthetic zeolite F-9 (supplied by Wako Pure Chemical Industries, Ltd.);
* basic activated alumina (for column chromatography use, produced by Wako Pure Chemical Industries, Ltd.).

### 2) Treatment method

The ozone-gas-treated semi-purified product (200 mL) was applied to a column where any one of the adsorbents described in the above item 1) (10 g) was packed in a ϕ 2 cm glass column, and the semi-purified product was flowed through at a 1.2 mL/min of treating velocity (space velocity SV = 5.0 (H⁻¹)). After cutting off the initial eluate (5 mL), the eluate was recovered.

### (4) Measurement

For the recovered eluate and the ozone-gas-treated semi-purified product collected as a sample, fluorescence intensity at exciting wavelength 254 nm and fluorescence wavelength 450 to 700 nm, and at exciting wavelength 365 nm and fluorescence wavelength 450 to 700 nm were measured by the same equipment and method as in Experimental Example 1-(4). Further, UV absorbance at wavelength 200 to 400 nm was also measured using a spectrophotometer.

### (5) Results

The obtained results are shown in Table 9. In Table 9, the absorbance at 254 to 400 nm shows the maximum value of the absorbance at 254 to 400 nm. Further, the fluorescence intensity shows the maximum value of the fluorescence intensity under the above-described measurement conditions as a quinine sulfate equivalent.

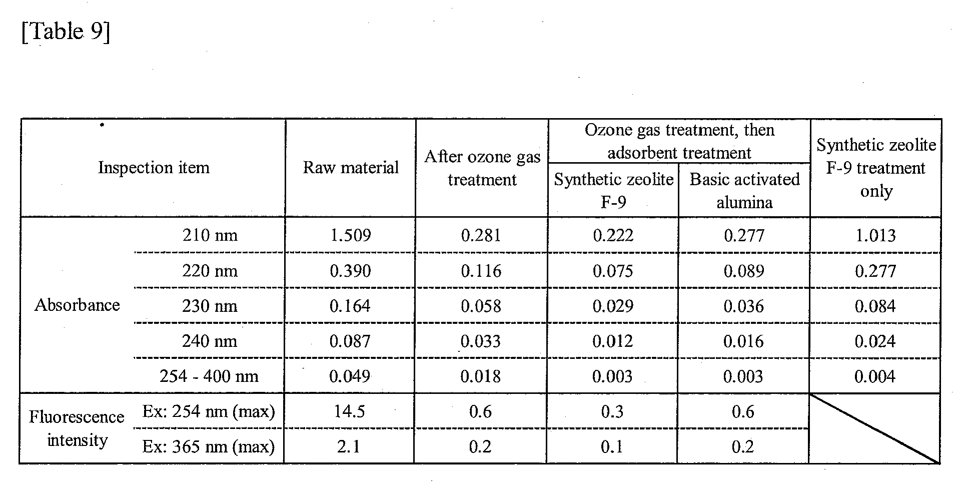

As obvious from the results of Table 9, the followings could be understood. That is, by only the treatment using an adsorbent (synthetic zeolite F-9), reduction of absorbance was very slight, and purification to a quality suitable for use as a mobile phase for HPLC could not be performed. On the other hand, by treating with ozone gas and subsequently treating with an adsorbent, absorbance was remarkably reduced, and a highly purified product which was usable as a mobile phase for HPLC could be obtained.

In addition, it can be understood that even when Y-type zeolite (synthetic zeolite HS-320) or a basic adsorbent (basic activated alumina) is used as an adsorbent, absorbance and fluorescence intensity can be reduced, and removal effect for fluorescent substance can be obtained.

In particular, it can be understood that the highest purification effect can be obtained when synthetic zeolite F-9 is used as an adsorbent.

### Example 7

### (1) Raw material: 2,2,4-trimethylpentane (raw material for industrial use)

### (2) Treatment with ozone gas

1) Ozone generator: same to the one used in Example 1.
2) Treatment method:
   The raw material (200 mL) was poured into a 1 L glass container provided with an ozone gas aeration tube connected to the ozone generator and an exhaust gas discharging tube. Ozone gas was bubbled into the raw material under the following conditions using the ozone generator, while the material was stirred.

Ozone gas aeration conditions (ordinary pressure)
amount of ozone gas to be generated: 0.3 g/hr;
flow rate of gas: 1 L/min;
treatment temperature: 20°C;
treatment time: 2 minutes (amount of ozone gas aerated: 0.01 g/200 mL raw material).
Ozone concentration in the ozone gas when aerated under the above-described conditions was about 0.2335 vol % (about 5 g/Nm³).

### (3) Treatment with adsorbent

The ozone-gas-treated semi-purified product (200 mL) was applied to a column where synthetic zeolite F-9 (10 g) was packed in a ϕ 2 cm glass column, and the semi-purified product was flowed through at a 1.2 mL/min of treating velocity (space velocity SV = 5.0 (H⁻¹)). After cutting off the initial eluate (5 mL), the eluate was recovered.

### (4) Measurement

For the recovered eluate, UV absorbance at wavelength 200 to 400 nm was measured by using the same equipment and method as in Experimental Example 1-(4).

Aside from this, for the eluate obtained by only the above-described adsorbent treatment without carrying out the ozone gas treatment, UV absorbance was similarly measured.

### (5) Results

The obtained results are shown in Table 10. In Table 10, the absorbance at wavelength 254 to 400 nm shows the maximum value of the absorbance at 380 to 400 nm.

**[Table 10]**

| Inspection item | | Raw material | Zeolite treatment only | Ozone gas treatment, then zeolite treatment |
|---|---|---|---|---|
| Absorbance | 210 nm | 1.275 | 0.746 | 0.257 |
| | 220 nm | 0.360 | 0.181 | 0.084 |
| | 230 nm | 0.095 | 0.043 | 0.031 |
| | 254 - 400 nm | 0.019 | 0.004 | 0.003 |

As obvious from the results of Table 10, the followings could be understood. That is, also when 2,2,4-trimethylpentane was purified, by only the treatment using an adsorbent (synthetic zeolite F-9), reduction of absorbance was very slight, and a purified product to a quality suitable for use as a mobile phase for HPLC could not be obtained. On the other hand, by treating with ozone gas and subsequently treating with an adsorbent, absorbance was remarkably reduced, and a highly purified product which was usable for HPLC could be obtained.

Further, from the results of the above Examples 6 and 7, it can be understood that the purification method of the present invention is effective for purification of a saturated hydrocarbons such as 2,2,4-trimethylpentane and n-heptane.

### Example 8: Study of the purification method of the present invention using a simulated kerosene

Kerosene is expected to be used as a fuel of household fuel battery, however, it has been known that a small amount of sulfur compound mixed in a commercial kerosene impairs catalyst performance of the fuel battery.

Such sulfur compound includes dibenzothiophene, 4,6-dimethylbenzothiophene, 3-methylbenzothiophene, and the like.

And so, simulated kerosene were prepared, where each of the above-described 3 kinds of sulfur compounds was added to undecane which is one of main components (hydrocarbons having 9 to 15 carbon atoms) of kerosene, and these simulated kerosene were purified by the purification method of the present invention.

### (1) Simulated kerosene

Each of dibenzothiophene, 4,6-dimethylbenzothiophene and 3-methylbenzothiophene (all produced by Wako Pure Chemical Industries, Ltd.) was added to special grade of n-undecane (produced by Wako Pure Chemical Industries, Ltd.) so as to become 30 mg/L, respectively, to prepare simulated kerosenes (sulfur concentration: about 0.0022 w/w%).

### (2) Treatment with ozone gas

1) Ozone generator: same as the one used in Example 1.
2) Treatment method
   The raw material (200 mL) was poured into a 1 L glass container provided with an ozone gas aeration tube connected to the ozone generator and an exhaust gas discharging tube. Ozone gas was bubbled into the raw material under the following conditions using the ozone generator, while the simulated kerosene was stirred.

Ozone gas aeration conditions (ordinary pressure)
amount of ozone gas to be generated: 0.3 g/hr;
flow rate of gas: 1 L/min;
treatment temperature: 20°C;
treatment time: 4 minutes (amount of ozone gas aerated: 0.02 g/200 mL raw material).
Ozone concentration in the ozone gas when aerated under the above-described conditions was about 0.2335 vol % (about 5 g/Nm³).

### (3) Treatment with adsorbent

The ozone-gas-treated semi-purified product (200 mL) was applied to a column where synthetic zeolite F-9 (10 g) was packed in a ϕ 2 cm glass column, and the semi-purified product was flowed through at a 1.2 mL/min of treating velocity (space velocity SV = 5.0 (H⁻¹)). After cutting off the initial eluate (5 mL), the eluate was recovered.

### (4) Measurement

For the recovered eluate, UV absorbance at wavelength 200 to 400 nm was measured by using the same equipment and method as in Experimental Example 1-(4).

In addition, quality evaluation by three-dimensional fluorescence spectrum was carried out under the following conditions.

Equipment: fluorescence spectrophotometer (manufactured by Hitachi High-Technologies Corp., F-4500);

### Measurement conditions:

measurement mode: three dimensional;
measured exciting wavelength: 200 to 800 nm;
measured fluorescence wavelength: 200 to 800 nm;
scanning speed: 2400 nm/min.

### (5) Results

1) Measurement results of absorbance
   The measurement results of absorbance are shown in Table 11.

**[Table 11]**

| Inspection item | | Simulated kerosene | Purified product |
|---|---|---|---|
| Absorbance | 210 nm | 10 or more | 0.303 |
| | 220 nm | 10 or more | 0.109 |
| | 230 nm | 10 or more | 0.038 |
| | 240 nm | 10 or more | 0.014 |
| | 254 nm | 10 or more | 0.003 |
| | 300 nm | 1.088 | 0.000 |

As obvious from the results of Table 11, the followings could be understood. That is, when the simulated kerosene were purified by the purification method of the present invention where the ozone gas treatment and the adsorbent treatment were used in combination, absorbance considered to be caused by the impurities was remarkably reduced, and the added sulfur compounds and impurities contained in undecane could be removed.

### 2) Quality evaluation by UV spectrum

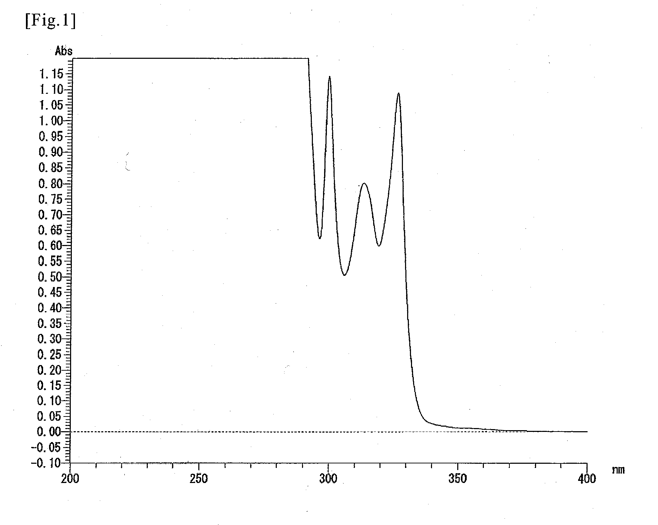

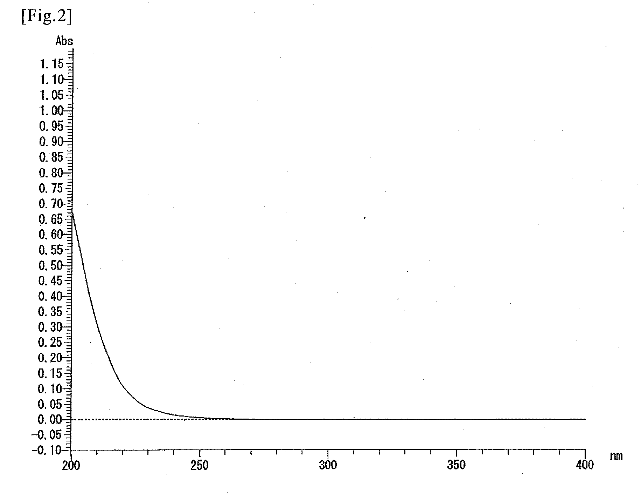

UV spectra in 200 to 400 nm are shown in Fig. 1 and Fig. 2. 1 shows a UV spectrum of simulated kerosene as a raw material. Fig. 2 shows a UV spectrum of purified product of the simulated kerosene. Further, in Fig. 1 and Fig. 2, the horizontal axis represents measured wavelength (nm) and the vertical axis represents absorbance (Ab), respectively.

As obvious from the results of Fig. 1 and Fig. 2, when the simulated kerosene prepared in the present Example were used, a UV absorption spectrum derived from the sulfur compounds and impurities in n-undecane as a raw material appeared (see Fig. 1). On the other hand, by purifying by the method of the present invention, this spectrum disappeared (see Fig. 2). From this, it could be understood that the sulfur compounds and impurities having UV absorption in the reagent could be removed effectively by the purification method of the present invention.

### 3) Quality evaluation by three-dimensional absorption spectra

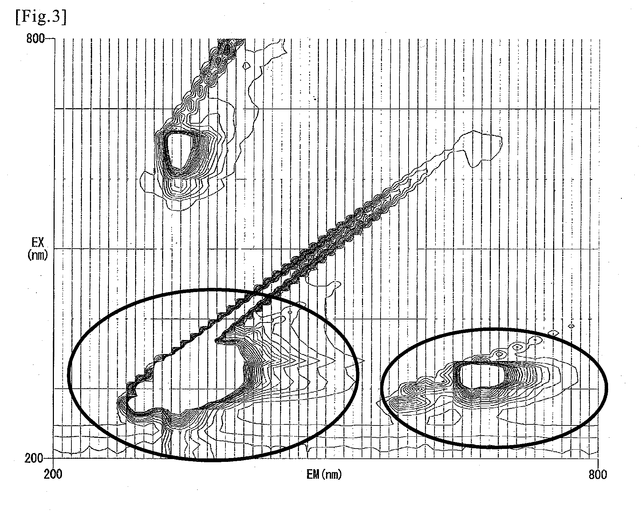

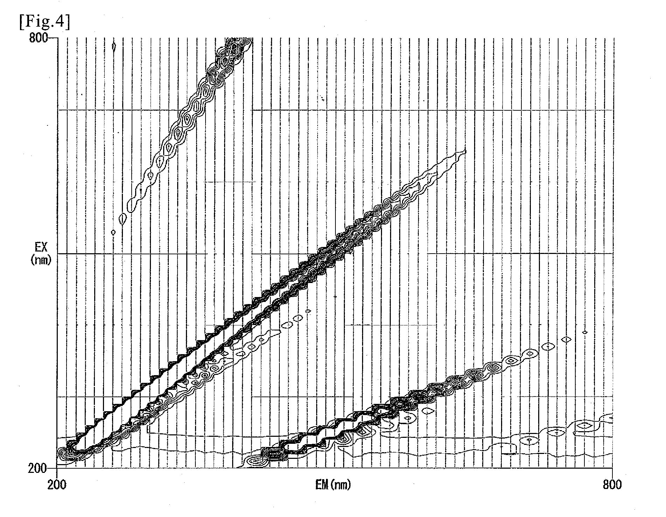

The evaluation results by three-dimensional fluorescence spectra are shown in Fig, 3 and Fig. 4. Fig. 3 shows a three-dimensional fluorescence spectrum of the simulated kerosene. Fig. 4 shows a three-dimensional fluorescence spectrum of the purified product of the simulated kerosene. Further, in Fig. 3 and Fig. 4, the horizontal axis represents fluorescence wavelength (EM (nm)), and the vertical axis represents exciting wavelength (EX (nm)), respectively

As obvious from Fig. 3, in the fluorescence spectra of the simulated kerosene, fluorescence spectra considered to be caused by the sulfur compounds and impurities in n-undecane as a raw material appeared (in Fig. 3, the places enclosed by the circles). On the other hand, by purifying the simulated kerosene by the purification method of the present invention, all of these spectra had been disappeared (see Fig. 4). From this, it can be understood that the sulfur compounds and impurities having fluorescence in n-undecane as a raw material could be removed effectively.

From the above-described results, it was indicated that the purification method of the present invention was very effective for removing sulfur compounds in kerosene.

### INDUSTRIAL APPLICABILITY

Since the purification method of the present invention can reduce amount of zeolite to be used to far smaller amount compared with that of the conventional method, for example, when 1,2,4-trichlorobenzene contaminated with PCB is treated by the purification method of the present invention, amount of zeolite contaminated with PCB produced by the purification can be dramatically reduced. Furthermore, since the purified product obtained by the purification method of the present invention is not required for further treatment such as distillation, a purified product having higher purity can be obtained more easily compared with the conventional method.

## Claims

1. A method for purifying a compound selected from a compound represented by the following general formula [1] or [2], a saturated aliphatic hydrocarbon having no substituent, or kerosene: 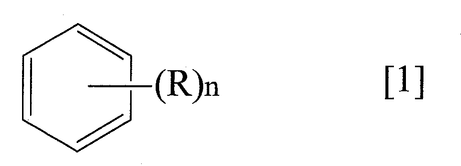
(R represents a halogen atom or a lower alkyl group having 1 to 3 carbon atoms, and n represents an integer of 1 to 5); 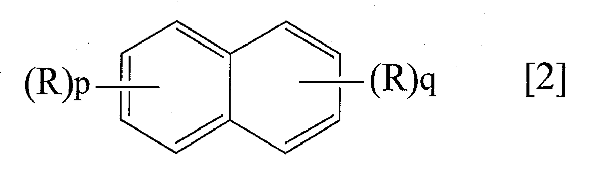
(R is the same as above, p and q independently represent an integer of 0 to 4, with the proviso that at least one ofp and q is not 0), comprising:
removing a impurity contained in said compound by
a first step comprising contacting said compound with ozone gas, and then
a second step comprising contacting said compound with an adsorbent selected from zeolite or a basic adsorbent.

2. The purification method according to claim 1, comprising only the first step and the second step.

3. The purification method according to claim 1 or 2, wherein the second step is a step where the said compound is contacted with zeolite.

4. The purification method according to any one of claims 1 to 3, wherein the compound represented by the general formula [1] is trichlorobenzene, chlorobenzene or ethylbenzene, the compound represented by the general formula [2] is 1-chloronaphthalene, 1-methylnaphthalene or 2-methylnaphthalene, and the saturated aliphatic hydrocarbon having no substituent is 2,2,4-trimethylpentane, cyclohexane, n-hexane, n-heptane, n-nonane or n-decane.

5. The purification method according to any one of claims 1 to 3, wherein the compound represented by the general formula [1] is 1,2,4-trichlorobenzene.

6. The purification method according to any one of claims 1 to 3, wherein the saturated aliphatic hydrocarbon having no substituent is 2,2,4-trimethylpentane or n-heptane.
